# EUROPEAN PATENT APPLICATION

(11) **EP 1 555 021 A1**
(43) Date of publication of application: **20.07.2005**
(21) Application number: 05290099.0
(22) Date of filing: 17.01.2005
(51) Int. Cl.: A61K 31/4439, A61K 31/427, A61K 31/366, A61K 45/06, A61P 35/00

(54) **Combinations of a mevalonate pathway inhibitor and a PPAR-gamma agonist for treating cancer**

(30) Priority: 16.01.2004 US 537235 P
(71) Applicant: National Health Research Institutes, Zhunan Town, Miaoli Country (TW)
(72) Inventor: Chuang, Shuang-En, Taipei (TW); Yao, Chih-Jung, Taipei (TW); Lai, Gi-Ming, Taipei (TW)
(74) Representative: Perin, Georges

(57) **Abstract**

This invention relates to the use of an effective amount of a mevalonate pathway inhibitor and an effective amount of a peroxisome proliferator-activated receptor γ agonist for the manufacture of a medicament for treating cancer, for the manufacture of a activity - enhanced composition for treating cancer and for the manufacture of a medicament for down-regulating the cell cycle and to pharmaceutical compositions.

## Description

This application relates to the use of an effective amount of a mevalonate pathway inhibitor and an effective amount of a peroxisome proliferator-activated receptor γ agonist for the manufacture of a medicament for treating cancer, for the manufacture of a activity - enhanced composition for treating cancer and for the manufacture of a medicament for down-regulating the cell cycle and to pharmaceutical compositions.

This application claims priority to U.S. provisional application number 60/537,235, filed on January 16, 2004, the content of which is incorporated herein by reference.

Statin family drugs, used to control hypercholesterolemia, block the mevalonate pathway of cholesterol synthesis. This pathway has also been known to play an important regulatory role in cellular proliferation and transformation. Recent studies indicate that statin family drugs inhibited tumor cell growth both *in vitro* and *in vivo* (see, e.g., Wong WW, et al., Leukemia, 2002, 16: 508-19). However, overall efficacy shown in these studies was not significant.

Troglitazone, a thiazolinedione type peroxisome proliferator-activated receptor γ (PPAR γ) agonist, is an anti-type II diabetes mellitus drug. Since PPAR γ agonists induce differentiation in cell lines derived from human malignancies, troglitazone has been investigated as a potential anticancer drug. Yet, results from clinical trials on various cancers were unsatisfactory (see, e.g., Kulke MH, et al., Cancer J., 2002, 8: 395-9; Burstein HJ, et al. Breast Cancer Res. Treat., 2003, 79: 391-7).

This invention is based on a surprising discovery that a PPAR γ agonist and a mevalonate pathway inhibitor or a mevalonate antagonist jointly exhibit synergistic effect on modulating several cycle cycle-regulating proteins. Specifically, the two active agents effectively lower the levels of cyclin dependent kinase-2 (CDK-2) and cyclin A, elevate the level of p27^{kip1}(a tumor suppressor), and diminish the phosphorylation of retinoblastoma (Rb) protein. Also surprisingly discovered is that the two active agents synergistically inhibit a number of cancer cells.

Thus, one aspect of this invention is a method of down-regulating the cell cycle by contacting cells with a mevalonate pathway inhibitor and a PPAR γ agonist or a mevalonate antagonist and a PPAR γ agonist. The mevalonate pathway inhibitor can be a 3-hydroxy-3-methylglutaryl-CoA (HMG-CoA) reductase inhibitor, such as a statin compound (e.g., lovastatin or simvastatin). The PPAR γ agonist can be troglitazone or pioglitazone. The mevalonate antagonist can be lovastatin or simvastatin.

Another aspect of this invention is a method of treating cancer (e.g., glioma, angiosarcoma, pancreatic cancer, prostate cancer, uterine cervical cancer, or lung cancer). The method includes administering to a subject in need thereof an effective amount of a PPAR γ agonist and an effective amount of a mevalonate pathway inhibitor or a mevalonate antagonist.

The above-mentioned method may also include administering to the subject to be treated an effective amount of an anticancer drug.

A further aspect of this invention is a method of enhancing efficacy of a chemotherapeutic anticancer agent. This method includes administering to a subject in need thereof an effective amount of the chemotherapeutic anticancer agent and an effective amount of a PPAR γ agonist. This method may also include administering to the subject an effective amount of a mevalonate pathway inhibitor or a mevalonate antagonist.

Also within the scope of this invention is a composition containing a mevalonate pathway inhibitor or a mevalonate antagonist, a PPAR γ agonist, and a pharmaceutically acceptable carrier thereof, as well as the use of such a composition for the manufacture of a medicament for treating cancer. The composition may further contain an anticancer drug.

Other features, objects, and advantages of the invention will be apparent from the description and the claims.

This invention features the use of an effective amount of a mevalonate pathway inhibitor and an effective amount of a peroxisome proliferator-activated receptor γ agonist for the manufacture of a medicament for treating cancer.

This invention also features the use of an effective amount of a mevalonate pathway inhibitor, an effective amount of a peroxisome proliferator-activated receptor γ agonist and an anticancer drug for the manufacture of a activity -enhanced composition for treating cancer.

This invention further features the use of an effective amount of a mevalonate pathway inhibitor and an effective amount of a peroxisome proliferator-activated receptor γ agonist for the manufacture of a medicament for down-regulating the cell cycle, preferably in vitro.

In the present application, the word "methods" hereafter refers to the above "uses".

This invention therefore features a method that includes administering to a subject in need thereof an effective amount of a PPARγ agonist and an effective amount of a mevalonate pathway inhibitor or a mevalonate antagonist to treat cancer or regulate the cell cycle. The term "mevalonate pathway inhibitor" refers to a compound that inhibits any enzyme of the mevalonate pathway (e.g., HMG-CoA reductase), thereby blocking the pathway. It can bind to the enzyme to exert the inhibitory activities or can inhibit the enzyme in an indirect manner in the mevalonate pathway. An example of such an inhibitor is a statin compound, e.g., lovastatin or simvastatin. The mevalonate pathway is well known in the art. See, e.g., *Biochemical pathways: An atlas of biochemistry and molecular biology;* Ed. Gerhard Michal, Wiley-Spektrum, 1998. A mevalonate antagonist is a compound that antagonizes effects exerted by mevalonate or lowers mevalonate levels. A PPARγ agonist refers to a substance that stimulates the activity of PPARγ. Examples of a suitable PPARγ agonist include, but are not limited to, troglitazone and pioglitazone.

The term "treating" as used herein refers to the application or administration of a composition including active agents to a subject, who has cancer, a symptom of cancer, or a predisposition toward cancer, with the purpose to cure, heal, alleviate, relieve, alter, remedy, ameliorate, improve, or affect the disease, the symptoms of the disease, or the predisposition toward the disease. "An effective amount" as used herein refers to the amount of each active agent which, upon administration with one or more other active agents to a subject in need thereof, is required to confer therapeutic effect on the subject. Effective amounts vary, as recognized by those skilled in the art, depending on route of administration, excipient usage, and the co-usage with other active agents.

The term "cancer" refers to cells having the capacity for autonomous growth, i.e., an abnormal state or condition characterized by rapidly proliferating cell growth. It is meant to include all types of cancerous growths or oncogenic processes, metastatic tissues or malignantly transformed cells, tissues, or organs, irrespective of histopathologic type or stage of invasiveness.

The term is meant to include all types of cancerous growths or oncogenic processes, metastatic tissues or malignantly transformed cells, tissues, or organs, irrespective of histopathologic type, or stage of invasiveness. Examples of cancers include, but are not limited to, carcinoma, heamatologic neoplasm, and sarcoma such as leukemia, sarcomas, osteosarcoma, lymphomas, melanoma, ovarian cancer, skin cancer, testicular cancer, gastric cancer, pancreatic cancer, renal cancer, breast cancer, prostate colorectal cancer, cancer of head and neck, brain cancer, esophageal cancer, bladder cancer, adrenal cortical cancer, lung cancer, bronchus cancer, endometrial cancer, nasopharyngeal cancer, cervical or hepatic cancer, or cancer of unknown primary site. In addition, cancer can be a drug resistance phenotype wherein cancer cells express P-glycoprotein, multidrug resistance-associated proteins, lung cancer resistance-associated proteins, breast cancer resistance proteins, or other proteins associated with resistance to anti-cancer drugs.

To practice the above-described method, the active agents can be applied at the same time or at different times. They can be administered orally, parenterally, by inhalation spray, or via an implanted reservoir. The term "parenteral" as used herein includes subcutaneous, intracutaneous, intravenous, intramuscular, intraarticular, intraarterial, intrasynovial, intrastemal, intrathecal, intralesional and intracranial injection or infusion techniques.

A pharmaceutical composition containing the two active agents and a pharmaceutically acceptable carrier can be used in the above-described method. The term "pharmaceutically acceptable carrier" refers to a carrier compatible with each active agent (and preferably, capable of stabilizing it) and not deleterious to the subject to be treated. For example, solubilizing agents, such as cyclodextrins, can be utilized as pharmaceutical excipients for delivery of the active agents. Examples of other carriers include colloidal silicon dioxide, magnesium stearate, cellulose, sodium lauryl sulfate, and D&C Yellow # 10.

A composition for oral administration can be any orally acceptable dosage form including, but not limited to, tablets, capsules, emulsions and aqueous suspensions, dispersions and solutions. Commonly used carriers for tablets include lactose and corn starch. Lubricating agents, such as magnesium stearate, are also typically added to tablets. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A sterile injectable composition (e.g., aqueous or oleaginous suspension) can be formulated according to techniques known in the art using suitable dispersing or wetting agents (such as, for example, Tween 80) and suspending agents. The sterile injectable preparation can also be a sterile injectable solution or suspension in a non-toxic parenterally acceptable diluent or solvent, e.g., 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g., synthetic mono- or diglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluent or dispersant, or carboxymethyl cellulose or similar dispersing agents.

An inhalation composition can be prepared according to techniques well known in the art of pharmaceutical formulation and can be prepared as solutions in saline, employing benzyl alcohol or other suitable preservatives, absorption promoters to enhance bioavailability, fluorocarbons, and/or other solubilizing or dispersing agents known in the art.

The above-mentioned cancer treatment method also includes administering to the subject having cancer an effective amount of an anticancer drug. The term "anticancer drug" refers to a drug to be used to treat cancer excluding mevalonate pathway inhibitors and PPARγ agonists, which have not been approved for cancer treatment. Examples of anticancer drugs include, but are not limited to, retinoids, non-steroidial (NSAIDs), cisplatin, taxol, doxorubicin, and 5- fluorouracil. The anticancer drug can be administered before or after the administration of a PPARγ agonist and a mevalonate pathway inhibitor (or a mevalonate antagonist).

This invention also features a method for enhancing efficacy of a chemotherapeutic anticancer drug. The method includes administering to a subject in need thereof an effective amount of the chemotherapeutic anticancer drug and an effective amount of a PPAR γ agonist. The term "chemotherapeutic anticancer drug" refers to a compound that treats cancer by selectively killing cancer cells, but not a compound that treats cancer by inducing differentiation of cancer cells (e.g., retinoids and non-steroidal anti-inflammatory drug). Examples of chemotherapeutic anticancer drugs include cisplatin, taxol, doxorubicin, and fluorouracil. This method may also include administering to the subject an effective amount of a mevalonate pathway inhibitor (a mevalonate antagonist). To practice this method, the active agents can be applied at the same time or at different times. They can be administered orally, parenterally, by inhalation spray, or via an implanted reservoir.

A suitable *in vitro* assay can be used to preliminarily evaluate the efficacy of combinations of active agents in modulating the levels of certain cell cycle-regulating proteins (e.g., CDK-2, cyclin A, Rb, E2F1, and p27^{kip1}) and inhibiting growth of cancer cells. The combination index (CI) is calculated according to the median effect equation shown in Chou, T. C. et al. Adv. Enzyme Regul., 1984, 22: 27-55. Of note, a CI represents the combination effect, such as, synergism, antagonism or addition, of two or more drugs. When the CI is lower than 1, the combination effect is synergistic; when the CI is equal to 1, the combination effect is additive; and when the CI is higher than 1, the combination effect is antagonistic.

Combinations of active agents can further be screened for their efficacy in treating caner by *in vivo* assays. For example, a combination of active agents can be injected into an animal (e.g., a mouse model) and its therapeutic effects are then accessed. Based on the results, an appropriate dosage range and administration route can also be determined.

The mevalonate pathway inhibitor, the mevalonate antagonist, the PPAR γ agonist, the anticancer drugs, and the chemotherapeutical anticancer drug used in this application are commercially available or can be synthesized by a method known in the art.

Without further elaboration, it is believed that the above description has adequately enabled the present invention. The following specific examples are, therefore, to be construed as merely illustrative, and not limitative of the remainder of the disclosure in any way whatsoever. All of the publications and U.S. provisional application No. 60/537,235, cited herein are hereby incorporated by reference in their entirety.

### Synergistic effect of lovastain and troglitazone on inhibiting human glioblastoma cells:

DBTRG 05MG cells (human glioblastoma cells) were treated for 6 days with 0.5 µM of lovastatin (Clabiochem) alone and 10 µM of troglitazone (Clabiochem) alone. Cell numbers were then measured by the sulforhodamine assay (see, e.g., Rubinstein, L. V. et al., J. Natl. Cancer Institute, 1990, 82: 113). The results show that the cell numbers decreased by 9.7% and 11%, respectively. By contrast, when DBTRG 05MG cells were treated simultaneously for 6 days with both lovastatin (0.5 µM) and troglitazone (10 µM), the cell number decreased by 82%.

The same cells were also treated with combinations of lovastatin and troglitazone at various ratios. The combination index values were calculated. All of these values were lower than 1, indicating synergistic effect.

In addition, microscopic observations also confirmed the synergistic effect of lovastain and troglitazone on inhibiting human glioblastoma cells.

### Synergistic effect of lovastain and troglitazone on other cancer cells

Similarly, lovastatin alone, troglitazone alone, and a combination of lovastatin and troglitazone were tested against a number of other cell lines, i.e., C6 (rat glioma cell line), CL1-0, CL1-5, and CL1-5F4 (human lung cancer cell line), MIA-PaCa2 (human pancreatic cancer cell line), Hela (human uterine cervical cancer cell line), Hep G2 (human hepatoblastoma cell line), PC3 (human prostate cancer cell line), SVR (murine endothelial cell line), and MS1 (murine endothelial cell line). The results show that the combination synergistically inhibited the tested cell lines.

### Synergistic effect of lovastain and troglitazone on regulating the cell cycle

Cell cycle-regulating proteins, cyclin-dependent kinase 2 (CDK-2), cyclin A, Rb protein, and p27^{kip1} were tested to determine the effect of lovastain and troglitazone on regulating the cell cycle. Note that p27^{kip1} is a universal CDK inhibitor and can modulate the drug resistance of cancer cells (Lloyd, R. V., Am. J. Pathol. 1999, 154: 313-323). Rb binds to many proteins, including several important gene regulatory proteins. Its binding capacity depends on its state of phosphoration. When Rb is dephosphorated, it binds to a set of regulatory proteins (e.g., E2F-1) that favor cell proliferation; and when phosphorated, it releases these proteins, thereby allowing them to act.

DBTRG 05MG cells were treated for 3 days with lovastatin (1µM) alone, toglitazone (10 µM) alone, and a combination of lovastatin (1 µM) and toglitazone (10 µM). The concentrations of cyclin-dependent kinase 2 (CDK-2), cyclin A, p27^{kip1}, Rb protein, and E2F-1 were determined by the Western blotting assay. Briefly, whole cell lysates were prepared with a radioimmunoprecipitation buffer supplemented with protease inhibitors (Sigma). The lysates were subjected to a sodium dodecylsulfate-polyacrylamide gel electrophoresis and transferred to polyvinylidene difluoride membranes (Bio-Rad) by eletroblotting. After having been blocked for 1 hour at room temperature, the membranes were probed with a primary antibody overnight at 4°C and then with a horseradish peroxidase-conjugated secondary antibody for 1 hour. The immune complexes were visualized using the Luminol Reagent (Santa Cruz), according to the protocol provided by the manufacturer. The primary antibodies to detect CDK-2 (sc-163), cyclin A (sc-239), E2F-1 (sc-251), and Rb (sc-102) were purchased from Santa Cruz Biotechnology, the primary antibody to detect p27^{kip1} (#610241) was purchased from BD Transduction Laboratories, and the primary antibody to detect phosphor-Rb (Ser807/811) was purchased from Cell Signaling Technology.

The results show that either lavostatin alone or togalitazone alone induced little or no change of the levels of CDK-2, cyclin A, RB, p27^{kip1}, Rb, and E2F-1. By contrast, the combination of lavostatin and togalitazone significantly lowered the levels of CDK-2, cyclin A, and E2F-1, elevated that of p27^{kip1}, and almost completely dephosphorated Rb at Ser807/811.

### Mevalonolacetone-pathway dependent effect of lovastatin and troglitazone

DBTRG 05MG cells were treated for 6 days with lovastatin (0.5 µM), troglitazone (10 µM), and mevalonolacetone (100 µM), the downstream product of HMV-Co reductase. Cell numbers were measured by the sulforhodamine assay (see, e.g., Rubinstein, L. V. et al., J. Natl. Cancer Institute, 1990, 82: 113). The result shows that the addition of mevalonolacetone significantly attenuated the synergistic effect of lovastatin and troglitazone.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions. Thus, other embodiments are also within the scope of the following claims.

## Claims

1. Use of an effective amount of a mevalonate pathway inhibitor and an effective amount of a peroxisome proliferator-activated receptor γ agonist for the manufacture of a medicament for treating cancer.

2. Use of an effective amount of a mevalonate pathway inhibitor, an effective amount of a peroxisome proliferator-activated receptor γ agonist and an anticancer drug, for the manufacture of an activity -enhanced composition for treating cancer.

3. Use of an effective amount of a mevalonate pathway inhibitor and an effective amount of a peroxisome proliferator-activated receptor γ agonist, for the manufacture of a medicament for down-regulating the cell cycle.

4. The use of claim 1,2 or 3 wherein the peroxisome proliferator-activated receptor γ agonist is troglitazone.

5. The use of claim 1,2 or 3 wherein the peroxisome proliferator-activated receptor γ agonist is pioglitazone.

6. The use of any of claims 1 to 5 wherein the mevalonate pathway inhibitor is a 3-hydroxy-3-methylglutaryl-CoA reductase inhibitor.

7. The use of any of claims 1 to 5 wherein the mevalonate pathway inhibitor is a 3-hydroxy-3-methylglutaryl-CoA reductase inhibitor which is a statin compound.

8. The use of any of claims 1 to 5 wherein the mevalonate pathway inhibitor is lovastatin.

9. The use of any of claims 1 to 5 wherein the mevalonate pathway inhibitor is simvastatin.

10. The use of any of claims 1 to 5 wherein the mevalonate pathway inhibitor is a a mevalonate antagonist.

11. The use of any of claims 1 to 10 wherein the cancer is glioma, angiosarcoma, lung cancer, pancreatic cancer, prostate cancer, uterine cervical cancer, or lung cancer.

12. The use of any of claims 2 and 4 to 11 wherein the anticancer drug is a chemotherapeutic anticancer agent.

13. A pharmaceutical composition comprising a mevalonate pathway inhibitor, a peroxisome proliferator-activated receptor γ agonist, and a pharmaceutically acceptable carrier thereof.

14. The pharmaceutical composition of claim 13, further comprising an anticancer drug.

15. The pharmaceutical composition of any of claims 13 and 14 wherein the peroxisome proliferator-activated receptor γ agonist is troglitazone.

16. The pharmaceutical composition of any of claims 13 and 14 wherein the peroxisome proliferator-activated receptor γ agonist is pioglitazone.

17. The pharmaceutical composition of any of claims 13 to 16 wherein the mevalonate pathway inhibitor is a 3-hydroxy-3-methylglutaryl-CoA reductase inhibitor.

18. The pharmaceutical composition of any of claims 13 to 16 wherein the mevalonate pathway inhibitor is a 3-hydroxy-3-methylglutaryl-CoA reductase inhibitor which is a statin compound.

19. The pharmaceutical composition of any of claims 13 to 16 wherein the mevalonate pathway inhibitor is lovastatin.

20. The pharmaceutical composition of any of claims 13 to 16 wherein the mevalonate pathway inhibitor is simvastatin.

21. The pharmaceutical composition of any of claims 13 to 16 wherein the mevalonate pathway inhibitor is a a mevalonate antagonist.

22. A method of down-regulating the cell cycle, comprising contacting cells preferably in vitro with a mevalonate pathway inhibitor and a peroxisome proliferator-activated receptor γ agonist or a mevalonate antagonist and a peroxisome proliferator-activated receptor γ agonist.

23. The method of claim 22, wherein the mevalonate pathway inhibitor is a statin compound.

24. The method of claim 23, wherein the statin compound is lovastatin or simvastatin.

25. The method of claim 24, wherein the peroxisome proliferator-activated receptor γ agonist is troglitazone or pioglitazone.
